(19) Europäisches Patentamt
European Patent Office
Office européen des brevets

(11) EP 3 554 426 B1

(12) FASCICULE DE BREVET EUROPEEN

(45) Date de publication et mention de la délivrance du brevet:
**06.01.2021 Bulletin 2021/01**

(21) Numéro de dépôt: **17821707.1**

(22) Date de dépôt: **11.12.2017**

(51) Int Cl.:
***A61F 2/40*** *(2006.01)*

(86) Numéro de dépôt international:
**PCT/FR2017/053487**

(87) Numéro de publication internationale:
**WO 2018/109341 (21.06.2018 Gazette 2018/25)**

(54) **PROCÉDÉ DE CONSTRUCTION D'UNE GAMME DE COMPOSANTS HUMÉRAUX POUR PROTHÈSES ARTICULAIRES DE L'ÉPAULE**

VERFAHREN ZUR HERSTELLUNG EINER REIHE VON HUMERUSKOMPONENTEN FÜR SCHULTERGELENKPROTHESEN

METHOD FOR CONSTRUCTING A RANGE OF HUMERAL COMPONENTS FOR SHOULDER JOINT PROSTHESES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.12.2016 FR 1662380**

(43) Date de publication de la demande:
**23.10.2019 Bulletin 2019/43**

(73) Titulaire: **Move-Up**
**26100 Romans (FR)**

(72) Inventeur: **GEAIS, Laurent**
**26100 Romans (FR)**

(74) Mandataire: **Chevalier, Renaud Philippe et al**
**Cabinet Germain & Maureau**
**BP 6153**
**69466 Lyon Cedex 06 (FR)**

(56) Documents cités:
**US-A- 5 725 592**
**US-A1- 2004 143 335**
**US-A1- 2015 250 601**
**US-A1- 2016 270 854**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 554 426 B1

## Description

La présente invention se rapporte au domaine des prothèses articulaires de l'épaule, et plus particulièrement au domaine des composants huméraux courts pour prothèses articulaires de l'épaule. L'état de la technique le plus proche est donné par le document US 5725592 A, qui divulgue un procédé de construction d'une gamme de composants huméraux destinés à être introduits dans une partie proximale de l'humérus.

La présente invention concerne notamment un procédé de construction d'une gamme de composants huméraux pour prothèses articulaires de l'épaule. La présente invention trouve application lors d'opérations chirurgicales de reconstruction de l'épaule.

Il est particulièrement souhaitable pour une prothèse articulaire de permettre la restitution des mobilités de l'articulation qu'elle remplace.

Dans le cas particulier d'une prothèse articulaire d'épaule, les divers bras de levier observés au niveau de l'articulation remplacée - c'est-à-dire les distances entre les centres de rotation et les points d'application des efforts au niveau des insertions musculaires correspondantes - doivent pouvoir être restitués avec la plus grande précision possible. Ces distances sont définies entre la forme endocanalaire du composant huméral de la prothèse et le centre de la surface articulaire.

Pour répondre notamment à l'objectif de conservation des divers bras de levier, le composant huméral, destiné à être introduit dans la partie proximale de l'humérus, doit présenter une forme endocanalaire adaptée au plus grand nombre possible de morphotypes de la population.

Or, les gammes de composants huméraux selon l'art antérieur ne permettent généralement de s'adapter que très partiellement aux diverses morphologies des patients, dont les tailles varient fortement.

En outre, le temps nécessaire pour développer une gamme de composants huméraux selon les procédés de construction connus est encore particulièrement important, car il est nécessaire de reprendre les calculs et la conception pour chaque taille de composants huméraux constituant la gamme. Il s'agit d'un processus itératif long et complexe, nécessitant un temps de marquage important.

Il est encore particulièrement souhaitable pour un composant huméral d'assurer un blocage angulaire en rotation optimale, afin d'éviter tous descellements, une fois disposé dans la partie proximale de l'humérus.

Or, la stabilité en rotation des composants huméraux selon l'art antérieur peut être améliorée, en particulier pour les composants huméraux courts - c'est-à-dire typiquement d'une longueur inférieure à 100 mm.

L'état de la technique peut également être illustré par les enseignements des documents US 5 725 592 et US 2015/0250601 qui divulguent des procédés de construction de gammes de composants huméraux destinés à être introduits dans la partie proximale de l'humérus lors d'une opération chirurgicale reconstructrice de l'épaule.

Un des objets de l'invention est de permettre la conception d'une gamme de composants huméraux aptes à permettre la restitution des mobilités de l'articulation qu'ils remplacent, en particulier les divers bras de levier observés au niveau de l'articulation remplacée.

Un des objets de l'invention est de permettre la conception d'une gamme de composants huméraux présentant une forme endocanalaire adaptée au plus grand nombre possible de morphotypes de la population.

Un des objets de l'invention est de réduire le temps nécessaire pour développer une gamme de composants huméraux.

Un des objets de l'invention est de permettre la conception d'une gamme de composants huméraux courts aptes à assurer un blocage angulaire en rotation optimale, afin d'éviter tous descellements, une fois disposé dans la partie proximale de l'humérus.

Un ou plusieurs de ces objets sont remplis par le procédé selon l'invention.

Plus particulièrement, selon un premier aspect, l'invention se rapporte à un procédé de construction d'une gamme de composants huméraux destinés à être introduits dans la partie proximale de l'humérus lors d'une opération chirurgicale reconstructrice de l'épaule. Le procédé comporte les étapes suivantes :

- obtention d'un ensemble de données statistiques relatives à des variables aptes à permettre la caractérisation de la géométrie d'une partie proximale d'humérus, à partir de données morphométriques de parties proximales d'humérus appartenant à un échantillon représentatif d'une population ;
- détermination, à partir d'une distribution statistique, d'un ensemble de tailles composant la gamme de composants huméraux ;
- pour chacune desdites tailles, détermination d'une mesure pour chacune des variables, en fonction de l'ensemble de données statistiques ;
- pour chacune desdites tailles, production d'un composant huméral en fonction de la mesure de chacune des variables correspondant à ladite taille.

En mettant en oeuvre le procédé selon l'invention, il est possible de maîtriser le temps nécessaire au développement d'une gamme de composants huméraux.

En outre, il est aussi possible d'optimiser la gestion de la gamme, au moyen de la distribution statistique, et donc définir une taille de stock optimale au niveau de la production, mais également aussi dans les cliniques et hôpitaux.

En outre, la gamme étant optimisée en fonction d'informations relatives à un échantillon représentatif d'une population, la gamme offerte au chirurgien permet d'aider aux choix d'un composant huméral adapté.

Les variables aptes à permettre la caractérisation de la géométrie d'une partie proximale d'humérus comprennent par exemple une ou plusieurs des variables suivantes :

- un offset médial,
- un offset postérieur,
- un offset mécanique,
- une rétrotorsion / bi-épicondylienne,
- une rétrotorsion / postérieurs,
- un angle cervico-diaphysaire,
- un diamètre de la surface articulaire,
- une épaisseur de la surface articulaire,
- un encombrement antéro-postérieur et médio-latéral de chacune des sections transverses du composant huméral.

La distribution statistique peut être déterminée, pour chacune des variables, en fonction de la valeur moyenne et de l'écart-type, relatifs à ladite variable, dans l'ensemble de données statistiques.

Avantageusement, chaque composant huméral de la gamme a une longueur inférieure à sensiblement 100 mm.

Le procédé peut alors comporter une étape d'obtention d'au moins une valeur de référence d'un couple de torsion susceptible de provoquer un descellement d'un composant huméral installé dans une partie proximale d'humérus, et, dans lequel, la mesure pour chaque variable, pour chacune desdites tailles, est déterminée en fonction de l'ensemble de données statistiques et en fonction de ladite au moins une valeur de référence.

Pour chacune desdites tailles, la mesure pour chacune des variables, en fonction de l'ensemble de données statistiques, peut être déterminée en :

- obtenant un modèle tridimensionnel de composant huméral comportant des sections réparties dans différents plans, chaque section étant définie par un ensemble de paramètres géométriques ;
- déterminant, pour chacune des sections, l'ensemble de paramètres géométriques correspondant, en fonction de l'ensemble de données statistiques relatives aux variables aptes à permettre la caractérisation de la géométrie d'une partie proximale d'humérus ;
- déterminant, pour chacun desdits paramètres géométriques, au moyen d'une fonction d'interpolation, les valeurs dudit paramètre entre chaque section.

Pour chacune des sections, l'ensemble de paramètres géométriques correspondant, peut être déterminé en fonction de l'ensemble de données statistiques relatives aux variables aptes à permettre la caractérisation de la géométrie d'une partie proximale d'humérus, et en fonction de ladite au moins une valeur de référence.

Les sections sont par exemple de forme sensiblement octogonale, chaque section étant pourvues de congés de rayons paramétrables, les valeurs des rayons paramétrables étant choisies en fonction de ladite au moins une valeur de référence.

Les sections octogonales permettent ainsi de passer de section stabilisante - c'est-à-dire à très faible rayon - à une section distale sensiblement en forme quasi-circulaire afin de ne pas entrer en contact avec la diaphyse.

Ainsi, il est possible de réduire considérablement les risques liés aux contacts du composant huméral avec la diaphyse, à savoir les problèmes de déviation des contraintes, plus couramment désignés par "stress-shielding" en anglais, et la survenue de douleurs en bout de tige.

Grâce au procédé de construction selon l'invention, il est donc possible de construire une gamme de composants huméraux destinés à être introduits dans la partie proximale de l'humérus lors d'une opération chirurgicale reconstructrice de l'épaule, chaque composant huméral étant construit suivant le procédé de construction selon le premier aspect.

Selon un deuxième aspect, l'invention se rapporte à système pour construire une gamme de composants huméraux destinés à être introduits dans la partie proximale de l'humérus lors d'une opération chirurgicale reconstructrice de l'épaule.

Le système est notamment adapté à mettre en oeuvre le procédé selon le premier aspect. Le système comporte :

- une base de données comportant un ensemble de données statistiques relatives à des variables aptes à permettre la caractérisation de la géométrie d'une partie proximale d'humérus, à partir de données morphométriques de parties proximales d'humérus appartenant à un échantillon représentatif d'une population ;

- un configurateur apte à :

  - déterminer, à partir d'une distribution statistique, d'un ensemble de tailles composant la gamme de composants huméraux ;
  - pour chacune desdites tailles, déterminer une mesure pour chacune des variables, en fonction de l'ensemble de données statistiques ;
  - un outil de production configuré pour produire, pour chacune desdites tailles, un composant huméral en fonction de la mesure de chacune des variables correspondant à ladite taille.

La présente invention sera bien comprise et ses avantages ressortiront aussi à la lumière de la description qui va suivre, donnée uniquement à titre d'exemple non limitatif et faite en référence aux dessins annexés, dans lesquels :

- la figure 1 est un organigramme illustrant un procédé de construction, selon un mode de réalisation de l'invention ;
- la figure 2 est un schéma d'un système de construction, selon un mode de réalisation de l'invention ;
- la figure 3 est une vue schématique d'un modèle de cylindre métaphysaire et de sphère épiphyse, selon une construction de référence, représenté en superposition d'une vue anatomique d'un humérus typique ;
- la figure 4 est une vue schématique du modèle de cylindre métaphysaire et de sphère épiphyse, selon la construction de référence ;
- la figure 5 est une vue schématique, représentant sur une vue anatomique détaillée d'une sphère épiphyse typique, des variables relatives à ladite sphère épiphyse ;
- la figure 6 est une vue schématique, représentant sur une vue anatomique générale d'un humérus typique, des variables relatives audit humérus ;
- la figure 7 est un diagramme représentant le nombre de personnes, dans un échantillon de population, correspondant à différentes valeurs en mm d'offset médial à partir de données statistiques sur un échantillon de population ;
- la figure 8 est une vue tridimensionnelle schématique, issue d'une simulation par éléments finis, sur laquelle est représenté le couple de torsion susceptible de provoquer des descellements dudit composant huméral lorsque ce dernier est implanté dans un humérus ;
- la figure 9 est un diagramme représentant les fonctions d'interpolation pour les paramètres suivants : largeur médiale ML_Med, largeur latérale ML_Lat, largeur médiale totale ML_Med_Total et largeur latérale totale ML_Lat_Total ;
- la figure 10 est un diagramme représentant les fonctions d'interpolation pour les paramètres suivant : épaisseur médiale AP_Med, épaisseur latérale AP_Lat et épaisseur totale AP_Total ;
- la figure 11 est un diagramme représentant les fonctions d'interpolation pour les paramètres suivants: rayon médial des octogones R_Med et rayon latéral des octogones R_Lat ;
- la figure 12 est un schéma, représentant, dans un mode de réalisation selon l'invention, un modèle tridimensionnel de composant huméral pour prothèse articulaire (mesures exprimées en mm) ;
- la figure 13 est un schéma, représentant, dans un mode de réalisation selon l'invention, d'une section proximale du modèle tridimensionnel de composant huméral pour prothèse articulaire ;
- la figure 14 est un schéma, représentant, dans un mode de réalisation selon l'invention, d'une section distale du modèle tridimensionnel de composant huméral pour prothèse articulaire (mesures exprimées en mm) ;
- la figure 15 est un schéma, représentant, dans un mode de réalisation selon l'invention, la distribution et le positionnement relatif des sections du modèle tridimensionnel de composant huméral pour prothèse articulaire ;
- la figure 16a est une vue tridimensionnelle montrant un composant humoral de taille 1, obtenu au moyen du procédé selon l'invention ;
- la figure 16b est une vue tridimensionnelle montrant un composant humoral de taille 9, obtenu au moyen du procédé selon l'invention ;
- la figure 17 est une vue tridimensionnelle représentant une gamme de composants huméraux pour prothèses, obtenue au moyen du procédé selon l'invention.

La figure 1 illustre un procédé de construction 100, pour construire une gamme de composants huméraux pour prothèses, notamment la gamme visible à la figure 17. Les composants huméraux de la gamme sont destinés à une chirurgie reconstructrice de l'épaule.
Chaque composant huméral de la gamme comporte une partie endocanalaire destinée à être introduite dans la partie proximale de l'humérus. La gamme de composants huméraux comporte une pluralité de composants huméraux courts - c'est-à-dire typiquement d'une longueur inférieure à 100 mm - visibles sur la figure 17. Il est en effet avantageux de proposer des composants huméraux courts, car il est ainsi possible de faciliter les opérations chirurgicales de reprise.
Les différentes formes endocanalaires des composants huméraux de la gamme obtenue par la mise en œuvre du procédé laissent aux chirurgiens la possibilité de choisir un composant huméral dans ladite gamme, avec une probabilité élevée que ledit composant huméral soit adapté à la morphologie de la partie proximale de l'humérus du patient à opérer. Le recouvrement est typiquement de 95% de la population pour une gamme dessinée avec une taille 1 ayant les paramètres de $\mu$-2$\sigma$ et une taille 9 ayant les paramètres de $\mu$+2$\sigma$, comme détaillé ci-après dans la description.

Le procédé comporte une première étape 110 au cours de laquelle des données morphométriques $D_M$, correspondant à des variables $V_n$ aptes à caractériser des parties proximales d'humérus appartenant à un échantillon E représentatif d'une population, sont collectées.

À l'issue de l'étape 110, un ensemble $E_{VS}$ de données statistiques, relatives aux variables $V_n$, est obtenu.

Avantageusement, le procédé comporte une deuxième étape 120, optionnelle, au cours de laquelle des valeurs de références $V_{Er}$ de couples de torsion susceptibles de provoquer des descellements de composants huméraux, installés dans des parties proximales d'humérus appartenant à un échantillon E' représentatif d'une population sont obtenues.

Le procédé comporte une troisième étape 130 au cours de laquelle, à partir d'une distribution statistique $D_T$, un ensemble G de tailles $T_n$ composant la gamme de composants huméraux, est déterminé.

Le procédé comporte une quatrième étape 140 au cours de laquelle, pour chaque taille $T_n$ de l'ensemble G, on détermine une mesure $m_{iTn-n}$, pour chaque variable $V_n$, en fonction de l'ensemble $E_{VS}$ de données statistiques, et, avantageusement, en fonction des valeurs de références $V_{Er}$.

Le procédé comporte une cinquième étape 150 au cours de laquelle, la gamme de composants huméraux est obtenue, en produisant, pour chaque taille $T_n$ de l'ensemble G, un composant huméral en fonction des mesures $m_{iTn-n}$ relatives aux variables $V_n$ correspondant à ladite taille $T_n$.

On se réfère à la figure 2, sur laquelle est représenté schématiquement un système de construction, selon un mode de réalisation de l'invention. Le système est notamment adapté pour mettre en œuvre le procédé de construction 100, pour construire une gamme de composants huméraux pour prothèses.

Le système permet de faciliter les étapes de conception de la gamme de composants huméraux, en offrant notamment la possibilité de modifier et d'évaluer la géométrie de chaque composant huméral conçu selon les données morphométriques et l'expérience de chirurgiens.

Le système comporte une base de données 210 comportant les données morphométriques $D_M$, correspondant aux variables $V_n$ aptes à caractériser des parties proximales d'humérus appartenant à l'échantillon E.

Le système comporte encore un modèle 220 apte à décrire, pour chaque taille $T_n$ de l'ensemble G, les mesure $m_{iTn-n}$, pour chaque variable $V_n$, en fonction de l'ensemble $E_{VS}$ de données statistiques, et, avantageusement, en fonction des valeurs de références $V_{Er}$.

Le système comporte un configurateur 230, couplé à la base de données 210 et au modèle 220, et apte à définir, pour chaque taille $T_n$ de l'ensemble G, un composant huméral en fonction des mesures $m_{iTn-n}$ relatives aux variables $V_n$ correspondant à ladite taille $T_n$.

Le configurateur 230 peut avantageusement être couplé à un logiciel de conception assisté par ordinateur, afin de permettre par exemple de simuler, automatiquement, les composants huméraux de l'ensemble G, en fonction des données et lois morphométriques disponibles.

Le système comporte un outil de production 240, couplé au configurateur 230, configuré pour produire, pour chaque taille $T_n$ de l'ensemble G, un composant huméral en fonction des mesures $m_{iTn-n}$ relatives aux variables $V_n$ correspondant à ladite taille $T_n$.

Le système comporte avantageusement un outil de prise en compte de retours d'expérience 250 à collecter de nouveaux ensembles de données statistiques, et couplé au configurateur 230 de sorte à permettre à ce dernier de prendre en compte lesdits nouveaux ensembles de données statistiques.

On se réfère maintenant aux figures 3 à 6, sur lesquels est représentée une construction de référence pour partie proximale d'humérus. Une construction de référence pour partie proximale d'humérus est un modèle géométrique comprenant des objets géométriques, caractérisés par un ensemble de variables dont les valeurs permettent de décrire des géométries de parties proximales d'humérus.

Plus particulièrement, sur les figures 2 à 5, est représenté un modèle de cylindre métaphysaire 12 et de sphère épiphyse 14.

Dans cette construction de référence, le cylindre métaphysaire 12 est représenté par un cylindre des moindres carrés, typiquement compris entre les plans XX' et YY', orthogonaux à l'axe diaphysaire de l'humérus, et distants sensiblement respectivement de 65 mm et de 105 mm, par rapport au sommet de la surface articulaire C - aussi appelée point de Hinge, et noté C sur la figure 6.

La sphère épiphyse 14 est représentée par une sphère des moindres carrés passant par les points palpés sur la surface articulaire de la sphère épiphyse de l'humérus 10.

Sur les figures 4 et 5 sont également représentées des variables relatives au cylindre métaphysaire 12 et à la sphère épiphyse 14.

Sur les figures 3 à 6, plus particulièrement, sont représentés :

- l'offset médial $\Delta$M, correspondant à la distance entre le centre de la tête humérale - c'est-à-dire centre de la sphère des moindres carrés - et l'axe diaphysaire projetée dans le plan frontal ;

- l'offset postérieur $\Delta P$, c'est-à-dire la distance entre le centre de la tête humérale - c'est-à-dire le centre de la sphère des moindres carrés - et l'axe diaphysaire projetée dans le plan sagittal ;
- l'offset mécanique $O_{MC}$, c'est-à-dire la distance entre le centre de la tête humérale - c'est-à-dire le centre de la sphère des moindres carrés - et l'axe diaphysaire ; l'offset mécanique $O_{MC}$ peut en particulier être déterminé à partir de l'expression mathématique suivante:

$$O_{MC} = \sqrt{{O_{MD}}^2 + {O_P}^2}$$

- la rétrotorsion / bi-épicondylienne $\beta_1$, c'est-à-dire l'angle entre la projection dans le plan axial du col de l'humérus et la ligne bi-épicondylienne;
- la rétrotorsion / postérieurs $\beta_2$, c'est-à-dire l'angle entre l'axe du col de l'humérus et les deux points les plus postérieurs de la surface articulaire distale de l'humérus ;
- l'angle cervico-diaphysaire $\alpha$ entre l'axe diaphysaire de l'humérus et l'axe normal OO' ;
- le diamètre $D_{SA}$ de la surface articulaire, soit la distance entre le point C et le point D ;
- l'épaisseur $E_{SA}$ de la surface articulaire.

Aussi, selon l'exemple de construction de référence précédemment décrite, les variables V(n) de l'ensemble EVS de données statistiques considérées sont donc les suivantes :

- l'offset médial $O_{MD}$,
- l'offset postérieur $O_P$,
- l'offset mécanique $O_{MC}$,
- la rétrotorsion / bi-épicondylienne $\beta_1$,
- la rétrotorsion / postérieurs $\beta_2$,
- l'angle cervico-diaphysaire $\alpha$,
- le diamètre $D_{SA}$ de la surface articulaire,
- l'épaisseur $E_{SA}$ de la surface articulaire.

L'ensemble $E_{VS}$ de données statistiques, relatives à des variables $V_n$ morphologiques de parties proximales d'humérus appartenant à l'échantillon E, est ainsi déterminé, pour lesdites variables.

Dans un mode de réalisation, l'ensemble $E_{VS}$ de données statistiques, relatives à des variables $V_n$ morphologiques de parties proximales d'humérus appartenant à l'échantillon E, est ainsi déterminé notamment à partir d'informations, par exemple comprises dans une base de données, regroupant des valeurs obtenues pour lesdites variables $V_n$ lors d'études statistiques.

Ainsi, le diagramme illustré sur la figure 7, représentant le nombre de personnes correspondant à différentes valeurs en mm d'offset médial $O_{MD}$ peut être obtenu à partir de données statistiques sur l'échantillon E, regroupé dans une base de données.

Au cours de la deuxième étape 120 optionnelle, au cours de laquelle des valeurs de références $V_{Er}$ de couples de torsion susceptibles de provoquer des descellements de composants huméraux, installées de parties proximales d'humérus, sont déterminées.

La figure 8 représente, une vue tridimensionnelle schématique, issue d'une simulation par éléments finis, sur laquelle est représenté un couple de torsion $C_T$ susceptible de provoquer un descellement dudit composant huméral lorsquece dernier est implanté dans un humérus.

Ces données peuvent également être obtenues, expérimentalement, en utilisant une clé dynamométrique de sorte à déterminer lesdites valeurs de références sur les couples de torsion susceptibles de provoquer un descellement d'un composant huméral implanté dans la partie proximale d'un humérus.

Au cours de la troisième étape 130, l'ensemble G de tailles $T_{(n)}$, comprises dans la gamme de composants huméraux, est déterminé, en fonction de l'ensemble $E_{VS}$ de données statistiques, à partir de la distribution statistique $D_T$.

Une taille est une valeur arbitraire se référant à un ensemble de valeurs pour les différentes variables $V_n$, les dimensions diaphysaires étant prédominantes.

Dans un mode de réalisation, la distribution statistique $D_T$ est déterminée, pour chacune des variables $V_n$, en fonction de la valeur moyenne M et de l'écart-type $\sigma$, relatifs à ladite variable $V_n$, dans l'ensemble de données statistiques pour l'échantillon E.

Ainsi, pour chaque variable $V_n$ morphologique pour l'échantillon E, pour une gamme comportant 5 différentes tailles, la distribution statistique DT peut être la suivante :

| | |
|---|---|
| Taille 1 | valeur moyenne M - 2 x écart-type $\sigma$ |
| Taille 3 | valeur moyenne M - 1 x écart-type $\sigma$ |
| Taille 5 | valeur moyenne M |
| Taille 7 | valeur moyenne M + 1 x écart-type $\sigma$ |
| Taille 9 | valeur moyenne M + 2 x écart-type $\sigma$ |

Le tableau suivant regroupe les valeurs moyennes et les écarts type selon la distribution statistique DT précédemment décrite, pour chaque variable $V_n$ morphologique pour l'échantillon E, obtenues à partir de données statistiques collectées à partir d'études médicales et d'essais :

| | M | $\sigma$ | M-2x$\sigma$ | M+2x$\sigma$ |
|---|---|---|---|---|
| offset médial $O_{MD}$ | 6.2 mm | 1.9 mm | 2.4 mm | 10.0 mm |
| offset postérieur $O_P$ | 1.7 mm | 1.7 mm | -1.7 mm | 5.1 mm |
| offset mécanique $O_{MC}$ | 6.4 mm | 2.5 mm | 2.9 mm | 11.2 mm |
| rétrotorsion / bi-épicondylienne $\beta_1$ | 17.9° | 13.7° | -9.5° | 45.3° |
| rétrotorsion / postérieurs $\beta_2$ | 23° | 12.5° | -2° | 48° |
| angle cervico-diaphysaire $\alpha$ | 134.2° | 4.9° | 123.8° | 144.6° |
| diamètre $D_{SA}$ | 44.2 mm | 4.0 mm | 36.2 mm | 52.2 mm |
| épaisseur $E_{SA}$ | 16.4 mm | 1.7 mm | 13.0 mm | 19.8 mm |

On se réfère maintenant aux figures 12 à 15, sur lesquelles est représenté, dans un mode de réalisation, un modèle tridimensionnel de composant huméral pour prothèse articulaire.

Le modèle comporte des sections $S_n$ réparties dans différents plans $P_n$. Dans l'exemple représenté sur les figures 11 à 14, neuf sections $S_1..S_9$ sont réparties dans des plans $P_1..P_9$ parallèles, orthogonaux à l'axe vertical XX'. Les sections $S_1..S_9$ sont espacées, deux à deux, selon une distance constante, proportionnellle à la longueur totale du composant huméral. Six sections $S_{10}..S_{11}$ sont réparties dans des plans $P_{10}..P_{15}$. Le plan $P_{10}$ est incliné selon un angle $\alpha_{10}$ par rapport au plan $P_9$. Le plan $P_{11}$ est incliné selon un angle $\alpha_{11}$ par rapport au plan $P_{10}$. Le plan $P_{12}$ est incliné selon un angle $\alpha_{12}$ par rapport au plan $P_{11}$. Le plan $P_{13}$ est incliné selon un angle $\alpha_{13}$ par rapport au plan $P_{12}$. Le plan $P_{14}$ est incliné selon un angle $\alpha_{14}$ par rapport au plan $P_{13}$. Le plan $P_{15}$ est incliné selon un angle $\alpha_{15}$ par rapport au plan $P_{14}$

Dans un mode de réalisation les angles $\alpha_{10..15}$ sont tous sensiblement égaux. La section $S_{15}$ est représentée sur la figure 13, la section $S_1$, sur la figure 14. Les sections $S_n$ sont de forme sensiblement octogonale, pourvues de congés paramétrables. Chaque section $S_n$ peut être décrite par un ensemble de paramètres $P_{Sn}$ :

- un rayon latéral $RL_n$,
- un rayon médial $RM_n$,
- une distance latérale $MLM_n$,
- une distance latérale total $MLMT_n$,
- une distance médiale $MLL_n$,
- une distance médiale totale $MLLT_n$,
- une distance latérale $APL_n$,
- une distance médiale $APM_n$,
- une distance totale $APT_{n.}$

Aussi, pour chaque taille $T_{(n)}$ de l'ensemble G, l'ensemble de paramètres $P_{Sn}$ est déterminé, pour chacune des sections $S_n$, en fonction de l'ensemble Evs de données statistiques. Puis, pour chaque paramètre $P_{Sn}$ décrivant les sections $S_n$, les variations de la valeur dudit paramètre entre chaque plan $P_n$ sont interpolées.

Les figures 9 à 11 représentent diverses fonctions $F_{PSn}$ d'interpolation propres aux différents paramètres $P_{Sn}$, pour la taille 5. Les fonctions $F_{PSn}$ d'interpolation sont typiquement des fonctions d'interpolation du 3$^{e}$ degré (avec donc 2 points d'inflexion).

Dans un mode de réalisation avantageux, pour chaque taille $T_{(n)}$ de l'ensemble G, l'ensemble de paramètres

$P_{Sn}$ est déterminé, pour chacune des sections $S_n$, en fonction de l'ensemble $E_{VS}$ de données statistiques et en fonction des valeurs de références $V_{Er}$ des couples de torsions susceptibles de provoquer des descellements de composants huméraux, déterminées au cours de la deuxième étape 120.

Plus particulièrement, le rayon latéral $RL_n$ et le rayon médial $RM_n$ pour chacune des sections $S_n$ peuvent être déterminé en fonction des valeurs de références $V_{Er}$ des efforts couples de torsion susceptibles de provoquer des descellements de composants huméraux.

Ainsi, les valeurs du rayon latéral $RL_n$ et du rayon médial $RM_n$ pour chacune des sections $S_n$ sont par exemple déterminées en fonction des valeurs de références $V_{Er}$ et donc du besoin en stabilité rotatoire :

- plus la valeur du rayon latéral $RL_n$ et/ou du rayon médial $RM_n$ est faible, et plus ladite stabilité rotatoire est importante, ce qui est souhaitable pour la zone proximale du composant huméral ;
- plus la valeur du rayon latéral $RL_n$ et/ou du rayon médial $RM_n$ est importante, et plus ladite stabilité rotatoire est faible, ce qui est souhaitable pour la zone distale du composant huméral de sorte à faciliter l'insertion dans l'humérus et de limiter les problèmes de déviation des contraintes, plus couramment désignés par "stress-shielding" en anglais.

La gamme de composants huméraux est obtenue, en produisant, pour chaque taille $T_{(n)}$ de l'ensemble G, un composant huméral en fonction du modèle correspondant obtenu, comme décrit précédemment.

Après avoir mis en œuvre le procédé de construction, on obtient donc la gamme de composants huméraux courts adaptés aux morphologies des patients, la gamme de composants huméraux permettant aux chirurgiens de disposer, pour, chaque patient, d'un composant huméral court optimisé.

La figure 16a montre en particulier, un exemple d'un composant humoral de taille 1 (mesures exprimées en mm) obtenue au moyen du procédé selon l'invention. La figure 16b montre en particulier, un exemple d'un composant humoral de taille 9 (mesures exprimées en mm) obtenue au moyen du procédé selon l'invention.

## Revendications

1. Procédé de construction (100) d'une gamme de composants huméraux destinés à être introduits dans la partie proximale de l'humérus lors d'une opération chirurgicale reconstructrice de l'épaule, le procédé comportant les étapes suivantes :

   - obtention (110) d'un ensemble ($E_{VS}$) de données statistiques relatives à des variables aptes à permettre la caractérisation de la géométrie d'une partie proximale d'humérus, à partir de données morphométriques ($D_M$) de parties proximales d'humérus appartenant à un échantillon représentatif d'une population ;
   - détermination (130), à partir d'une distribution statistique, d'un ensemble (G) de tailles composant la gamme de composants huméraux ;
   - pour chacune desdites tailles, détermination (140) d'une mesure ($m_{iTn-n}$) pour chacune des variables, en fonction de l'ensemble de données statistiques ;
   - pour chacune desdites tailles, production (150) d'un composant huméral en fonction de la mesure de chacune des variables correspondant à ladite taille.

2. Procédé selon la revendication 1, dans lequel les variables aptes à permettre la caractérisation de la géométrie d'une partie proximale d'humérus comprennent une ou plusieurs des variables suivantes : un offset médial ($O_{MD}$), un offset postérieur ($O_P$), un offset mécanique ($O_{MC}$), une rétrotorsion / bi-épicondylienne ($\beta_1$), une rétrotorsion / postérieurs ($\beta_2$), un angle cervico-diaphysaire (CCD), un diamètre ($D_{SA}$) de la surface articulaire, une épaisseur ($E_{SA}$) de la surface articulaire, un encombrement antéro-postérieur et médio-latéral de chacune des sections transverses du composant huméral.

3. Procédé selon l'une quelconque des revendications précédentes, dans lequel la distribution statistique est déterminée, pour chacune des variables, en fonction de la valeur moyenne et de l'écart-type, relatifs à ladite variable, dans l'ensemble de données statistiques.

4. Procédé selon la revendication l'une quelconque des revendications précédentes, dans lequel chaque composant humérale de la gamme a une longueur inférieure à sensiblement 100 mm.

5. Procédé selon la revendication 4, comportant en outre une étape d'obtention (120) d'au moins une valeur de référence ($V_{Er}$) d'un couple de torsion susceptible de provoquer un descellement d'un composant huméral installé dans une partie proximale d'humérus ;

et, dans lequel, la mesure pour chaque variable, pour chacune desdites tailles, est déterminée (140) en fonction de l'ensemble de données statistiques et en fonction de ladite au moins une valeur de référence.

**6.** Procédé selon l'une quelconque des revendications précédentes, dans lequel pour chacune desdites tailles, la mesure ($m_{iTn-n}$) pour chacune des variables, en fonction de l'ensemble de données statistiques, est déterminée en :

- obtenant un modèle tridimensionnel de composant huméral comportant des sections ($S_n$) réparties dans différents plans ($P_n$), chaque section étant définie par un ensemble de paramètres géométriques ;
- déterminant, pour chacune des sections, l'ensemble de paramètres géométriques correspondant, en fonction de l'ensemble ($E_{VS}$) de données statistiques relatives aux variables aptes à permettre la caractérisation de la géométrie d'une partie proximale d'humérus ;
- déterminant, pour chacun desdits paramètres géométriques, au moyen d'une fonction d'interpolation, les valeurs dudit paramètre entre chaque section.

**7.** Procédé selon les revendications 5 et 6, dans lequel pour chacune des sections, l'ensemble de paramètres géométriques correspondant, est déterminé en fonction de l'ensemble ($E_{VS}$) de données statistiques relatives aux variables aptes à permettre la caractérisation de la géométrie d'une partie proximale d'humérus, et en fonction de ladite au moins une valeur de référence.

**8.** Procédé selon la revendication 7, dans lequel les sections sont de forme sensiblement octogonale, chaque section étant pourvues de congés de rayons paramétrables, les valeurs des rayons paramétrables étant choisies en fonction de ladite au moins une valeur de référence.

**9.** Système pour construire une gamme de composants huméraux destinés à être introduits dans la partie proximale de l'humérus lors d'une opération chirurgicale reconstructrice de l'épaule, le système comportant

- une base de données (210) comportant un ensemble ($E_{VS}$) de données statistiques relatives à des variables aptes à permettre la caractérisation de la géométrie d'une partie proximale d'humérus, à partir de données morphométriques ($D_M$) de parties proximales d'humérus appartenant à un échantillon représentatif d'une population ;
- un configurateur (230) apte à :
    - déterminer, à partir d'une distribution statistique, d'un ensemble (G) de tailles composant la gamme de composants huméraux ;
    - pour chacune desdites tailles, déterminer une mesure ($m_{iTn-n}$) pour chacune des variables, en fonction de l'ensemble de données statistiques ;
    - un outil de production (240) configuré pour produire, pour chacune desdites tailles, un composant huméral en fonction de la mesure de chacune des variables correspondant à ladite taille.

**Patentansprüche**

**1.** Verfahren zur Herstellung (100) einer Reihe von Humeruskomponenten, die zum Einsetzen in den proximalen Teil des Humerus bei einer rekonstruierenden chirurgischen Operation der Schulter bestimmt sind, wobei das Verfahren die folgenden Schritte aufweist:

- Erhalten (110) einer Menge ($E_{VS}$) statistischer Daten, die sich auf Variablen beziehen, die imstande sind, die Charakterisierung der Geometrie eines proximalen Humerusteils auf der Basis morphometrischer Daten ($D_M$) proximaler Humerusteile zu gestatten, die zu einem repräsentativen Panel einer Bevölkerung gehören;
- Bestimmen (130), auf der Basis einer statistischen Verteilung, einer Menge (G) von Größen, die die Reihe von Humeruskomponenten bilden;
- Bestimmen (140), für jede der Größen, eines Maßes ($m_{iTn-n}$) für jede der Variablen, in Abhängigkeit der Menge statistischer Daten;
- Herstellen (150), für jede der Größen, einer Humeruskomponente in Abhängigkeit von dem Maß von jeder der Variablen entsprechend der Größe.

**2.** Verfahren nach Anspruch 1, wobei die Variablen, die imstande sind, die Charakterisierung der Geometrie eines proximalen Humerusteils zu gestatten, eine oder mehrere der folgenden Variablen umfassen: ein mediales Offset

($O_{MD}$), ein posteriores Offset ($O_P$), ein mechanisches Offset ($O_{MC}$), eine Retrotorsion/biepikondylär ($\beta_1$), eine Retrotorsion/posterior ($\beta_2$), einen zervikodiaphysären Winkel (CCD), einen Durchmesser ($D_{SA}$) der Gelenkfläche, eine Dicke ($E_{SA}$) der Gelenkfläche, einen Platzbedarf antero-posterior und mediolateral vom jedem der Querschnitte der Humeruskomponente.

**3.** Verfahren nach einem der vorangehenden Ansprüche, wobei die statistische Verteilung für jede der Variablen in Abhängigkeit vom Mittelwert und von der Standardabweichung bestimmt wird, die sich auf die Variable beziehen, in der Menge der statistischen Daten.

**4.** Verfahren nach einem der vorangehenden Ansprüche, wobei jede Humeruskomponente der Reihe eine Länge von unter etwa 100 mm hat.

**5.** Verfahren nach Anspruch 4, aufweisend ferner einen Schritt des Erhaltens (120) von mindestens einem Referenzwert ($V_{Er}$) eines Torsionsmoments, das eine Lockerung einer in einem proximalen Humerusteil installierten Humeruskomponente verursachen könnte;
und wobei das Maß für jede Variable für jede der Größen in Abhängigkeit von der Menge statistischer Daten und in Abhängigkeit von dem mindestens einen Referenzwert bestimmt wird (140).

**6.** Verfahren nach einem der vorangehenden Ansprüche, wobei für jede der Größen das Maß ($m_{iTn-n}$) für jede der Variablen in Abhängigkeit von der Menge statistischer Daten bestimmt wird durch:

- Erhalten eines dreidimensionalen Humeruskomponentenmodells, das Querschnitte ($S_n$) aufweist, die in verschiedenen Ebenen ($P_n$) verteilt sind, wobei jeder Querschnitt von einer Menge geometrischer Parameter bestimmt wird;
- Bestimmen, für jeden der Querschnitte, die entsprechende Menge geometrischer Parameter in Abhängigkeit von der Menge ($E_{VS}$) statistischer Daten, die sich auf Variablen beziehen, die imstande sind, die Charakterisierung der Geometrie eines proximalen Humerusteils zu gestatten;
- Bestimmen, für jeden der geometrischen Parameter, mittels einer Interpolationsfunktion, der Werte des Parameters zwischen jedem Querschnitt.

**7.** Verfahren nach Anspruch 5 und 6, wobei für jeden der Querschnitte die entsprechende Menge geometrischer Parameter in Abhängigkeit von der Menge ($E_{VS}$) statistischer Daten bestimmt wird, die sich auf Variablen beziehen, die imstande sind, die Charakterisierung der Geometrie eines proximalen Humerusteils zu gestatten, und in Abhängigkeit von dem mindestens einen Referenzwert.

**8.** Verfahren nach Anspruch 7, wobei die Querschnitte etwa achteckig sind, wobei jeder Querschnitt mit parametrierbaren Radiusausrundungen versehen ist, wobei die Werte der parametrierbaren Radien in Abhängigkeit von dem mindestens einen Referenzwert ausgewählt werden.

**9.** System zur Herstellung einer Reihe von Humeruskomponenten, die zum Einsetzen in den proximalen Teil des Humerus bei einer rekonstruierenden chirurgischen Operation der Schulter bestimmt sind, wobei das System aufweist:

- eine Datenbank (210), aufweisend eine Menge ($E_{VS}$) statistischer Daten, die sich auf Variablen beziehen, die imstande sind, die Charakterisierung der Geometrie eines proximalen Humerusteils auf der Basis morphometrischer Daten ($D_M$) proximaler Humerusteile zu gestatten, die zu einem repräsentativen Panel einer Bevölkerung gehören;
- einen Konfigurator (230), der imstande ist:

  - auf der Basis einer statistischen Verteilung eine Menge (G) von Größen zu bestimmen, die die Reihe von Humeruskomponenten bilden;
  - für jede der Größen ein Maß ($m_{iTn-n}$) für jede der Variablen in Abhängigkeit von der Menge statistischer Daten zu bestimmen;
  - ein Produktionswerkzeug (240), das ausgelegt ist, um für jede der Größen eine Humeruskomponente in Abhängigkeit von dem Maß von jeder der Variablen entsprechend der Größe zu produzieren.

## Claims

1. A method for constructing (100) a range of humeral components intended to be introduced into the proximal portion of the humerus during a shoulder reconstruction surgery, the method including the following steps of:

   - obtaining (110) a set ($E_{VS}$) of statistical data relating to variables adapted to enable the characterization of the geometry of a humerus proximal portion, from morphometric data ($D_M$) of humerus proximal portions belonging to a representative sample of a population;
   - determining (130), from a statistical distribution, a set (G) of sizes composing the range of humeral components;
   - for each of said sizes, determining (140) a measurement ($m_{iTn-n}$) for each of the variables, according to the set of statistical data;
   - for each of said sizes, producing (150) a humeral component according to the measurement of each of the variables corresponding to said size.

2. The construction method according to claim 1, wherein the variables adapted to enable the characterization of the geometry of a humerus proximal portion comprise one or more of the following variables: a medial offset ($O_{MD}$), a posterior offset ($O_P$), a mechanical offset ($O_{MC}$), a retrotorsion / bi-epicondylar ($\beta_1$), a retrotorsion / posterior ($\beta_2$), a cervico-diaphysial angle (CCD), a diameter ($D_{SA}$)of the joint surface, a thickness ($E_{SA}$) of the joint surface, an anteroposterior and mediolateral bulk of each of the transverse sections of the humeral component.

3. The construction method according to any one of the preceding claims, wherein the statistical distribution can be determined, for each of the variables, according to the average value and the standard deviation, relating to said variable, in the set of statistical data.

4. The construction method according to any one of the preceding claims, wherein each humeral component of the range has a length substantially smaller than 100 mm.

5. The construction method according to claim 4, further including a step of obtaining (120) at least one reference value ($V_{Er}$) of a torsion torque likely to cause loosening of a humeral component installed in a proximal portion of the humerus,
   and wherein, the measurement for each variable, for each of said sizes, is determined (140) according to the set of statistical data and according to said at least one reference value.

6. The construction method according to any one of the preceding claims, wherein for each of said sizes, the measurement ($m_{iTn-n}$) for each of the variables, according to the set of statistical data, is determined by:

   - obtaining a three-dimensional model of the humeral component including sections ($S_n$) distributed in different planes ($P_n$), each section being defined by a set of geometric parameters;
   - determining, for each of the sections, the set of corresponding geometrical parameters, according to the set ($E_{VS}$) of statistical data relating to the variables adapted to enable the characterization of the geometry of a proximal portion of the humerus;
   - determining, for each of said geometrical parameters, by means of an interpolation function, the values of said parameter between each section.

7. The construction method according to claims 5 and 6, wherein for each of the sections, the set of corresponding geometrical parameters can be determined according to the set ($E_{VS}$) of statistical data relating to the variables adapted to enable the characterization of the geometry of a proximal portion of the humerus, and according to said at least one reference value.

8. The construction method according to claim 7, wherein the sections have a substantially octagonal shape, each section being provided with fillets with configurable radii, the values of the configurable radii being selected according to said at least one reference value.

9. A system for constructing a range of humeral components intended to be introduced into the proximal portion of the humerus during a shoulder reconstruction surgery, the system including:

   - a database (210) including a set ($E_{VS}$) of statistical data relating to variables adapted to enable the characterization of the geometry of a proximal portion of the humerus, from morphometric data ($D_M$) of proximal portions of the humerus belonging to a representative sample of a population;
- a configurator (230) adapted to:

  - determine, from a statistical distribution, a set (G) of sizes composing the range of humeral components;
  - for each of said sizes, determine a measurement ($m_{iTn-n}$) for each of the variables, according to the set of statistical data;

- a production tool (240) configured to produce, for each of said sizes, a humeral component according to the measurement of each of the variables corresponding to said size.

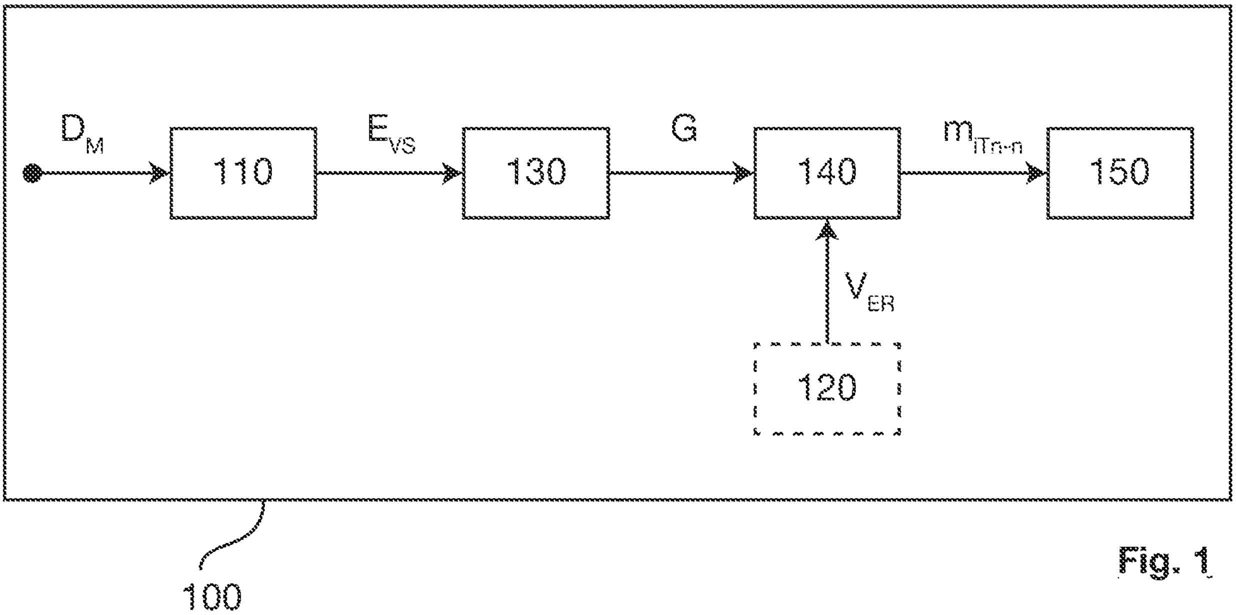
$D_M$
110
$E_{VS}$
130
G
140
$m_{iTn-n}$
150
$V_{ER}$
120
100

Fig. 1

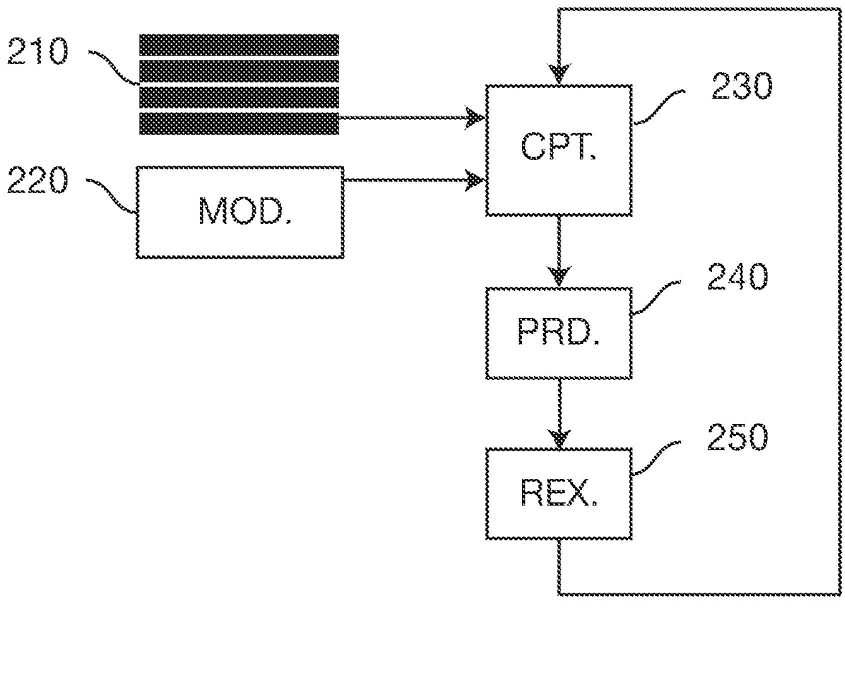
210
220
MOD.
CPT.
230
PRD.
240
REX.
250

Fig. 2

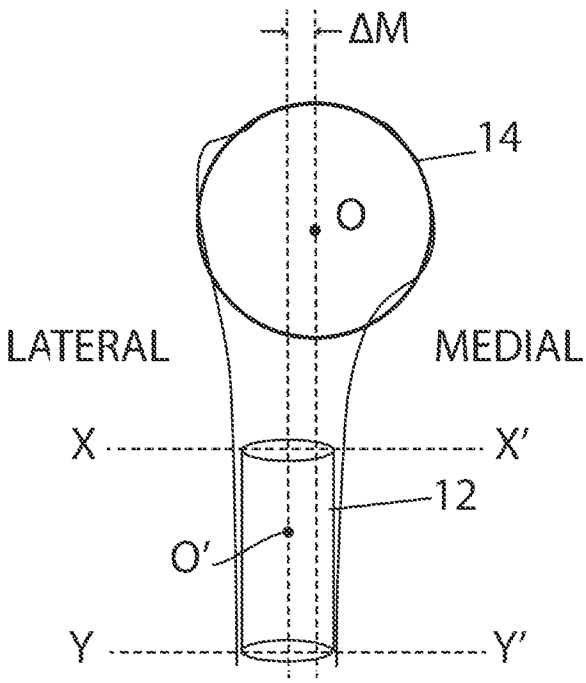
ΔM
14
O
LATERAL
MEDIAL
X
X'
12
O'
Y
Y'

Fig. 3

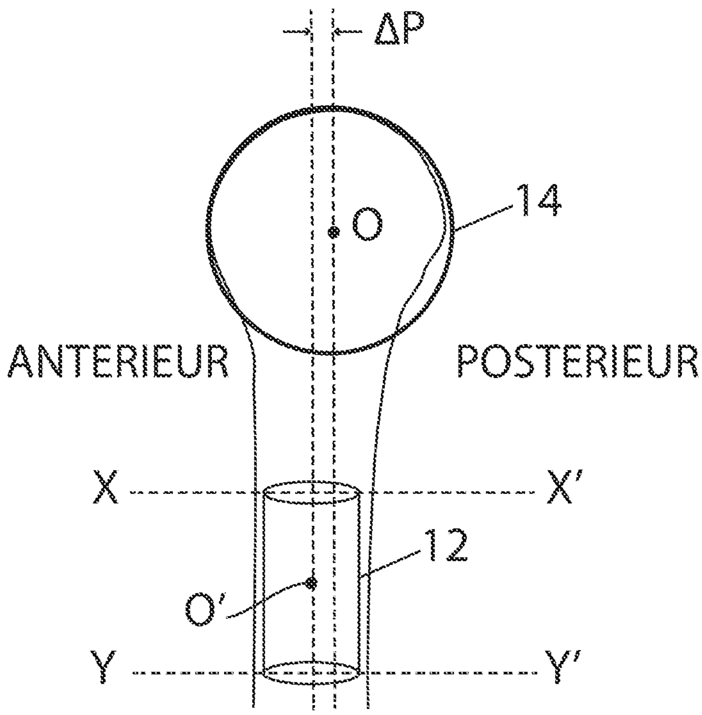
ΔP
14
O
ANTERIEUR
POSTERIEUR
X
X'
12
O'
Y
Y'

Fig. 4

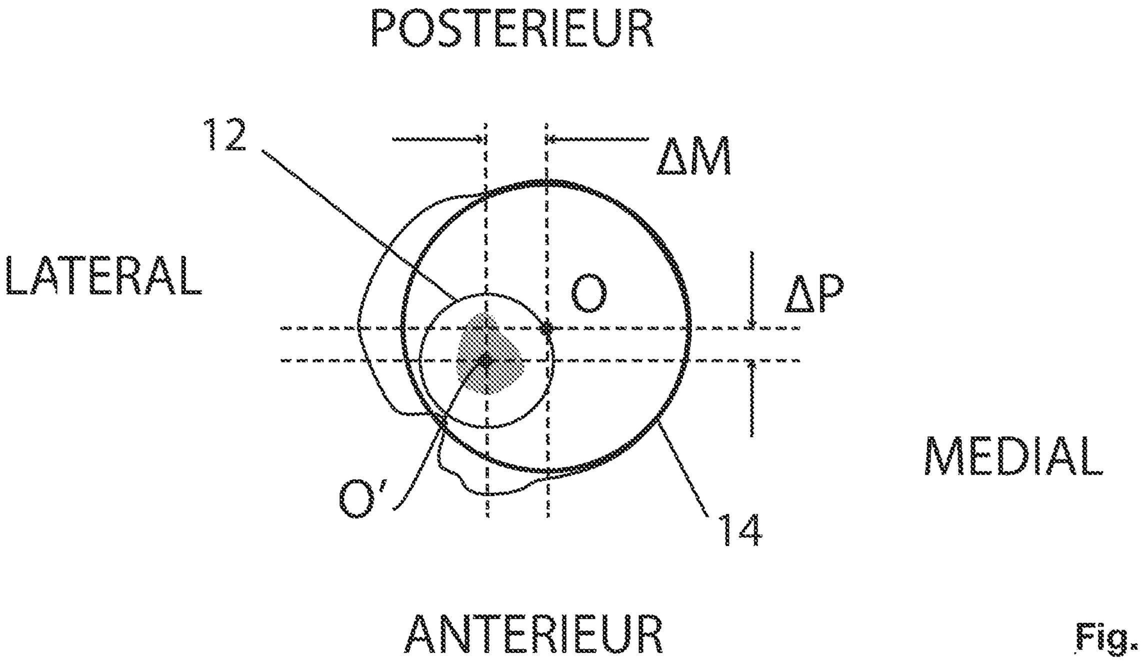
POSTERIEUR
12
ΔM
LATERAL
O
ΔP
MEDIAL
O'
14
ANTERIEUR

Fig. 5

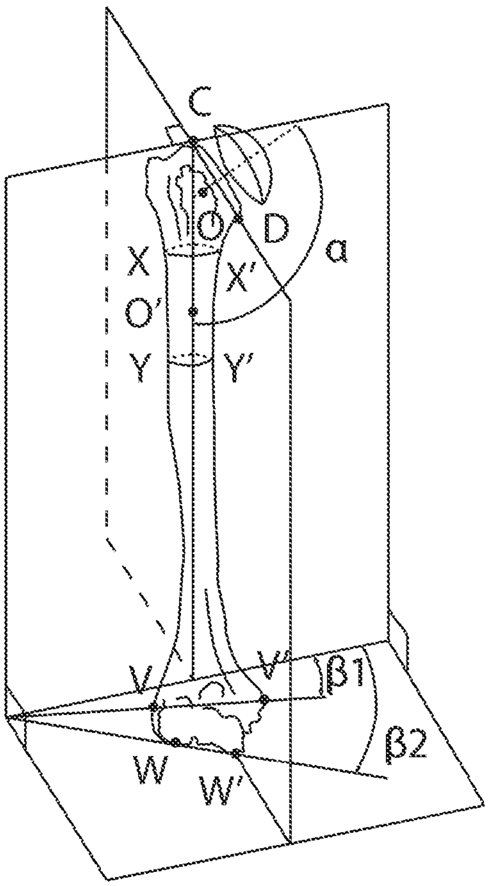
C
O
D
α
X
X'
O'
Y
Y'
V
V'
β1
β2
W
W'

Fig. 6

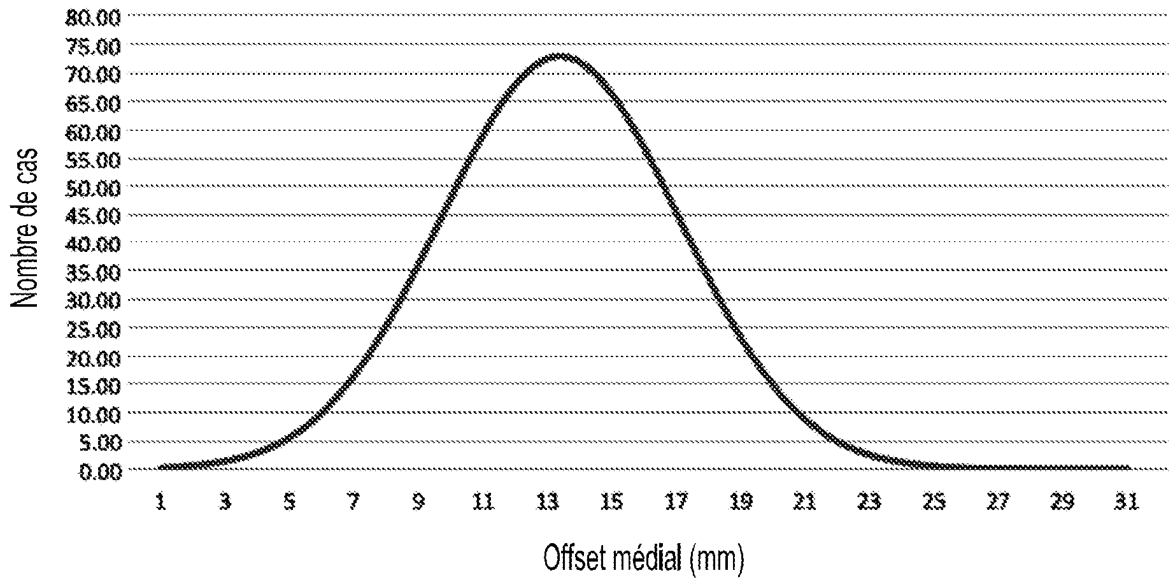
80.00
75.00
70.00
65.00
60.00
55.00
50.00
45.00
40.00
35.00
30.00
25.00
20.00
15.00
10.00
5.00
0.00
Nombre de cas
1
3
5
7
9
11
13
15
17
19
21
23
25
27
29
31
Offset médial (mm)

*Fig. 7*

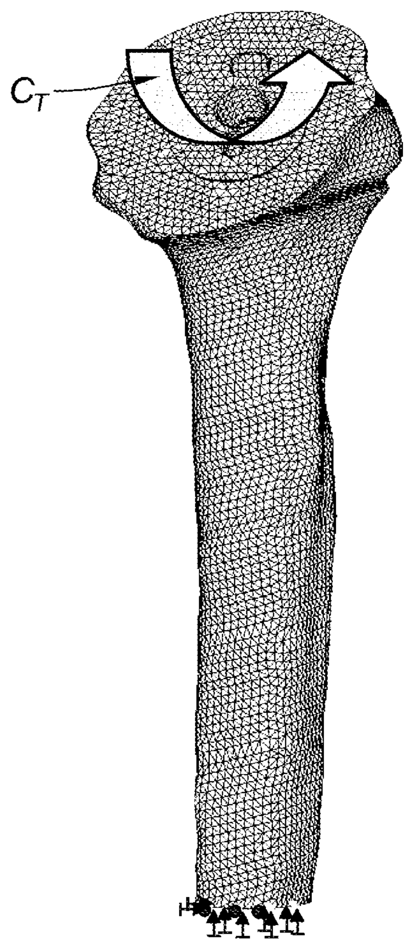
$C_T$

*Fig. 8*

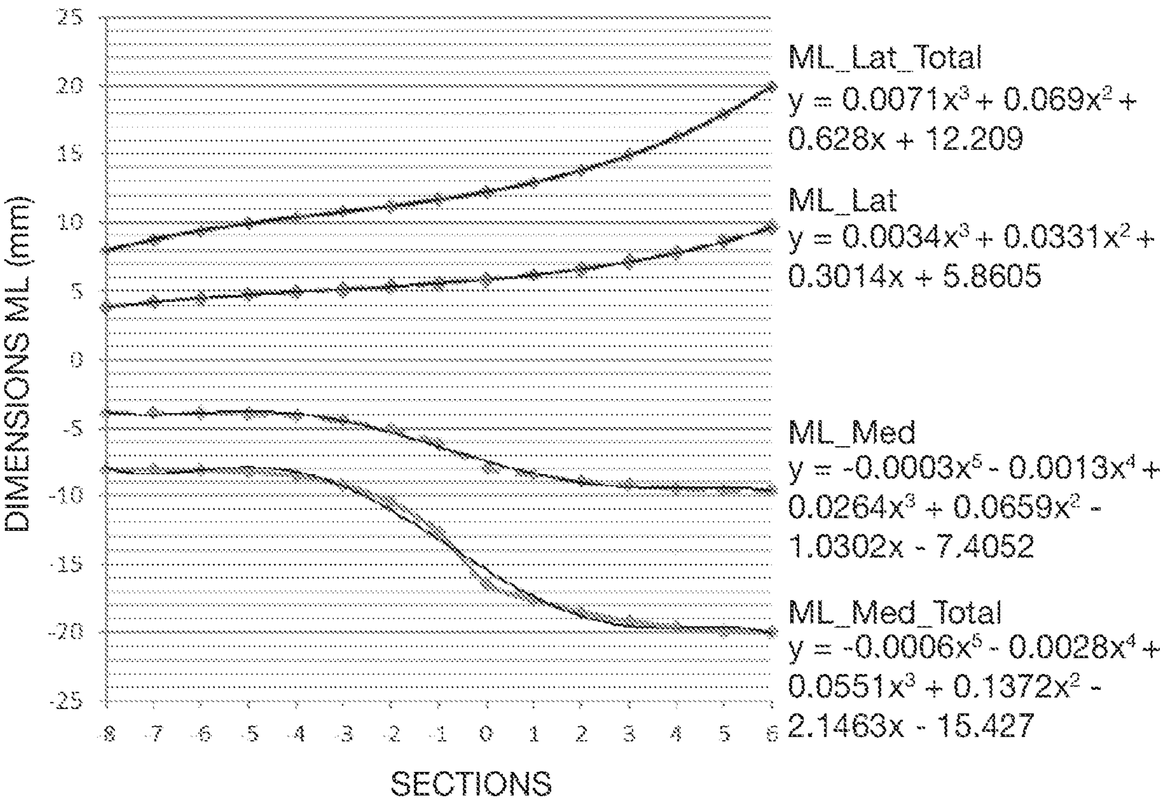
ML_Lat_Total
y = 0.0071x³ + 0.069x² + 0.628x + 12.209
ML_Lat
y = 0.0034x³ + 0.0331x² + 0.3014x + 5.8605
ML_Med
y = -0.0003x⁵ - 0.0013x⁴ + 0.0264x³ + 0.0659x² - 1.0302x - 7.4052
ML_Med_Total
y = -0.0006x⁵ - 0.0028x⁴ + 0.0551x³ + 0.1372x² - 2.1463x - 15.427
DIMENSIONS ML (mm)
SECTIONS
25
20
15
10
5
0
-5
-10
-15
-20
-25
-8 -7 -6 -5 -4 -3 -2 -1 0 1 2 3 4 5 6

Fig. 9

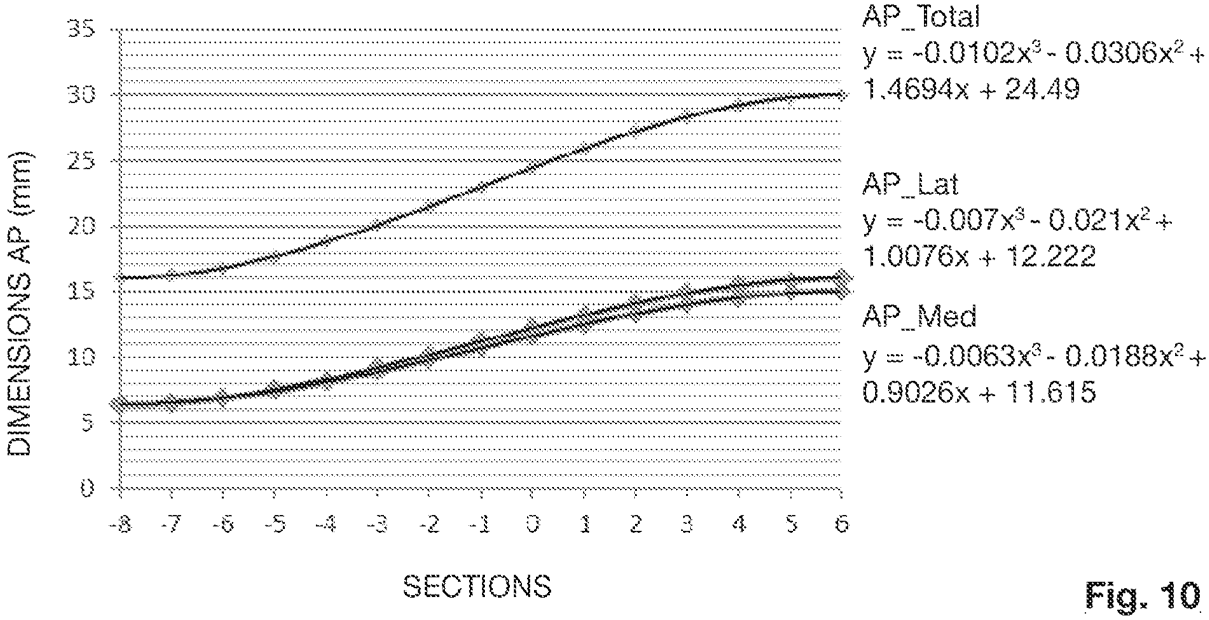
AP_Total
y = -0.0102x³ - 0.0306x² + 1.4694x + 24.49
AP_Lat
y = -0.007x³ - 0.021x² + 1.0076x + 12.222
AP_Med
y = -0.0063x³ - 0.0188x² + 0.9026x + 11.615
DIMENSIONS AP (mm)
SECTIONS
35
30
25
20
15
10
5
0
-8 -7 -6 -5 -4 -3 -2 -1 0 1 2 3 4 5 6

Fig. 10

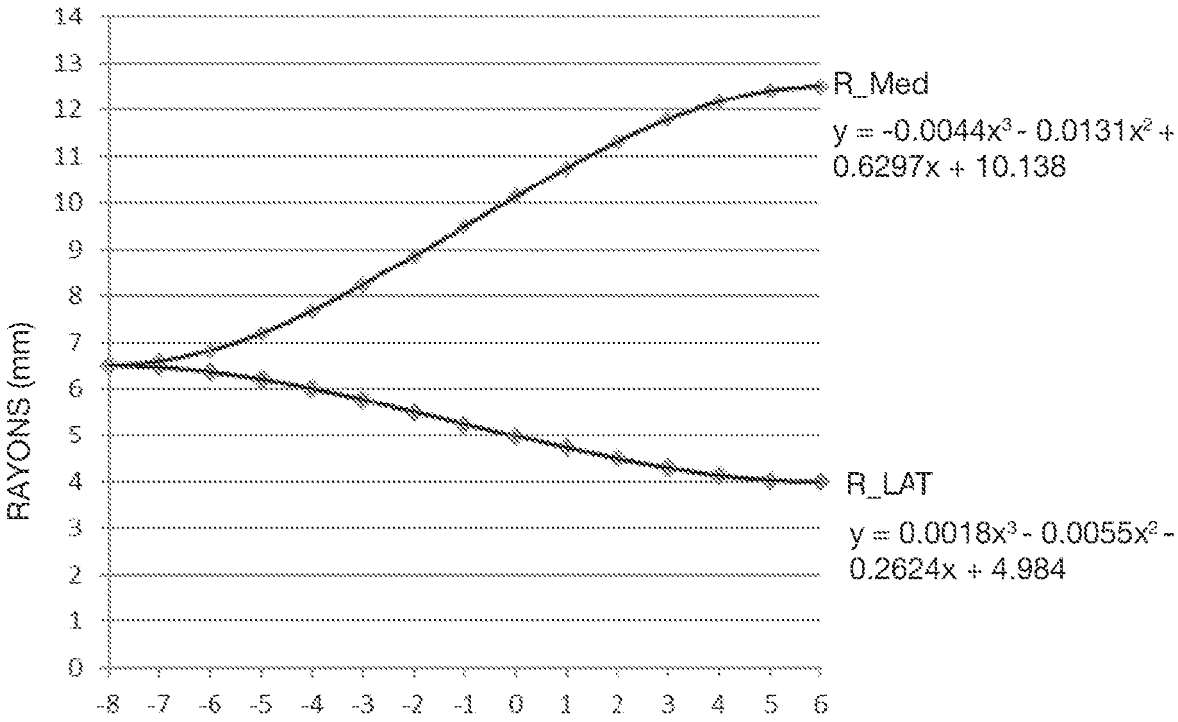
RAYONS (mm)
R_Med
y = -0.0044x³ - 0.0131x² + 0.6297x + 10.138
R_LAT
y = 0.0018x³ - 0.0055x² - 0.2624x + 4.984

Fig. 11

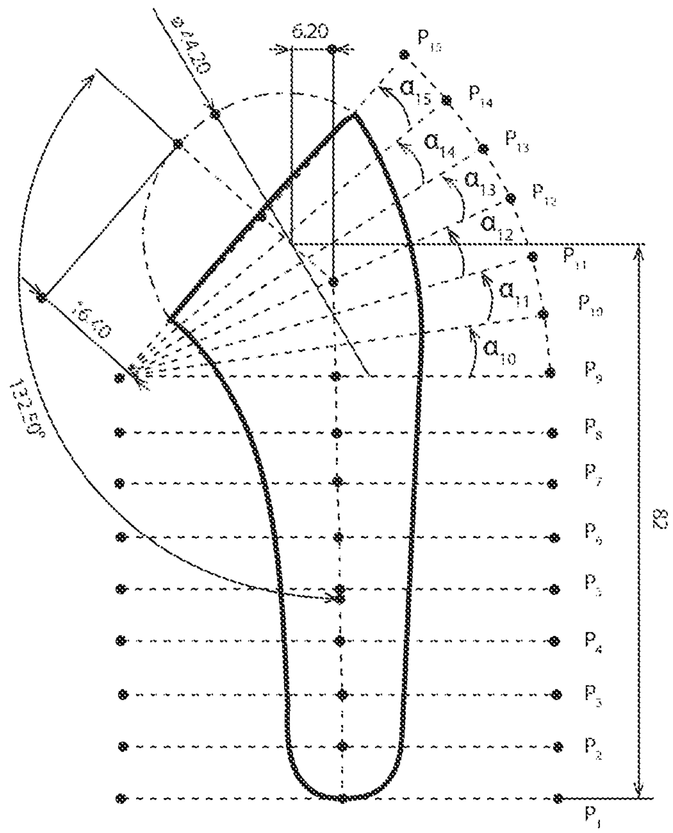
6.20
ø 44.20
16.00
132.50°
82

Fig. 12

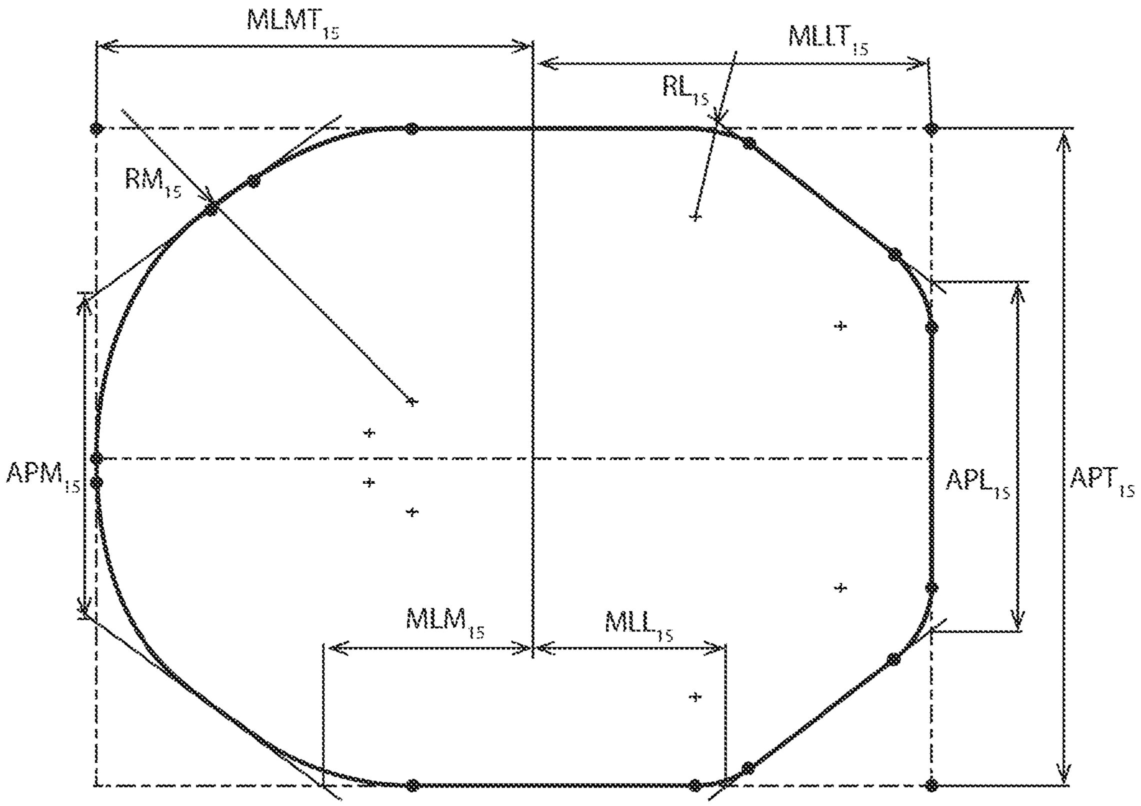
MLMT15
MLLT15
RL15
RM15
APM15
APL15
APT15
MLM15
MLL15

Fig. 13

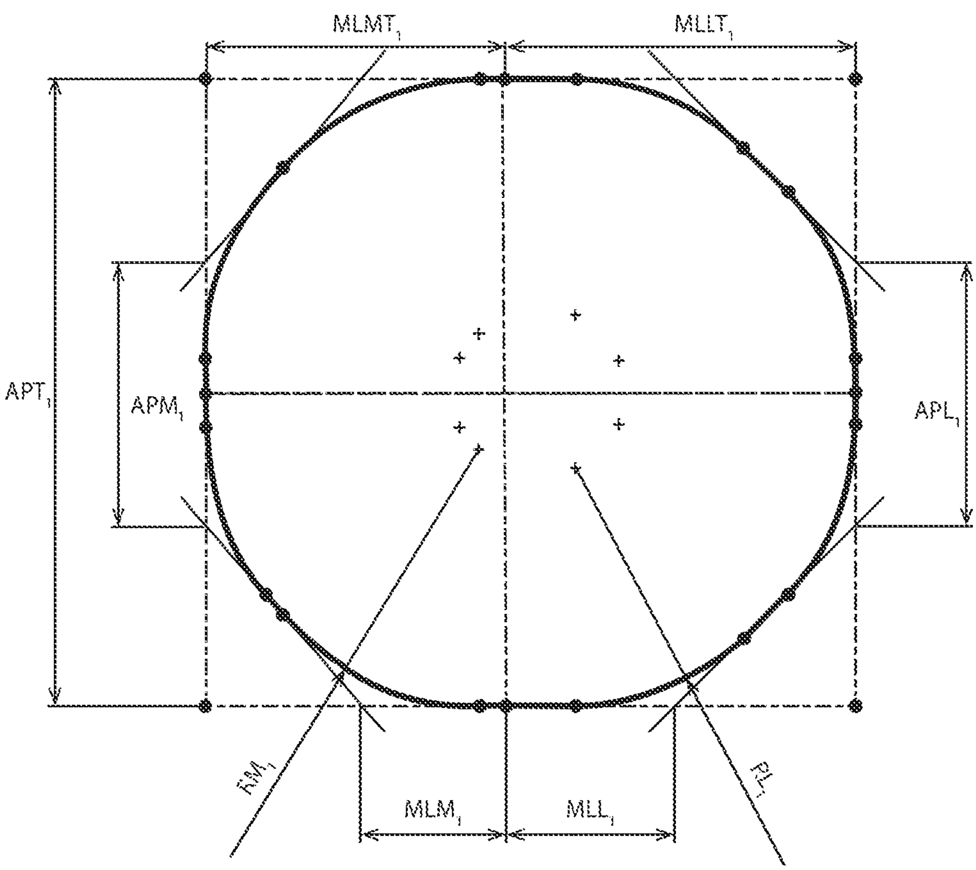
MLMT1
MLLT1
APT1
APM1
APL1
RM1
RL1
MLM1
MLL1

Fig. 14

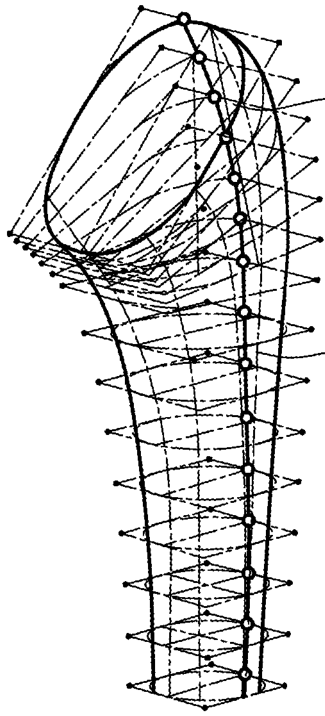


Fig. 15

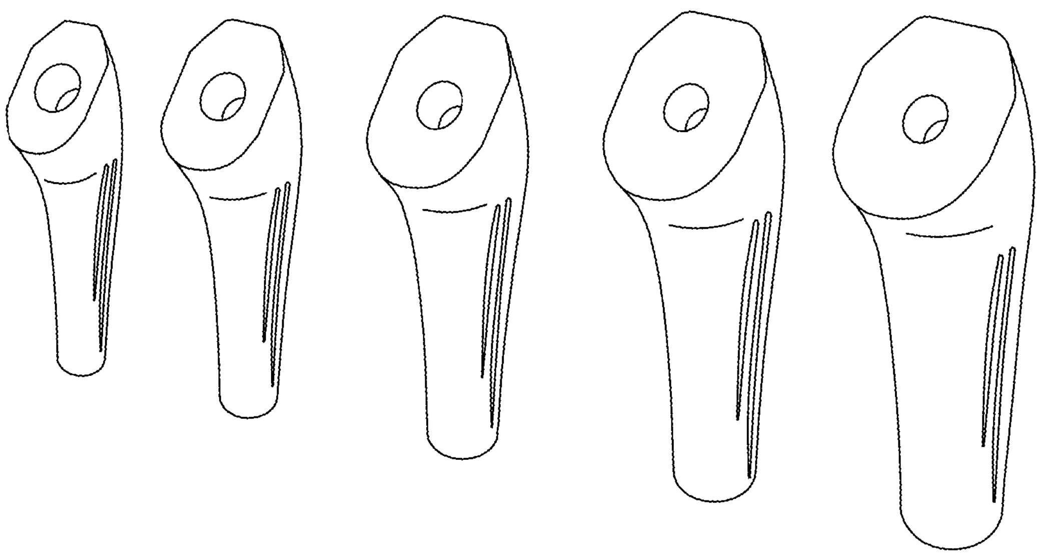

Fig. 17

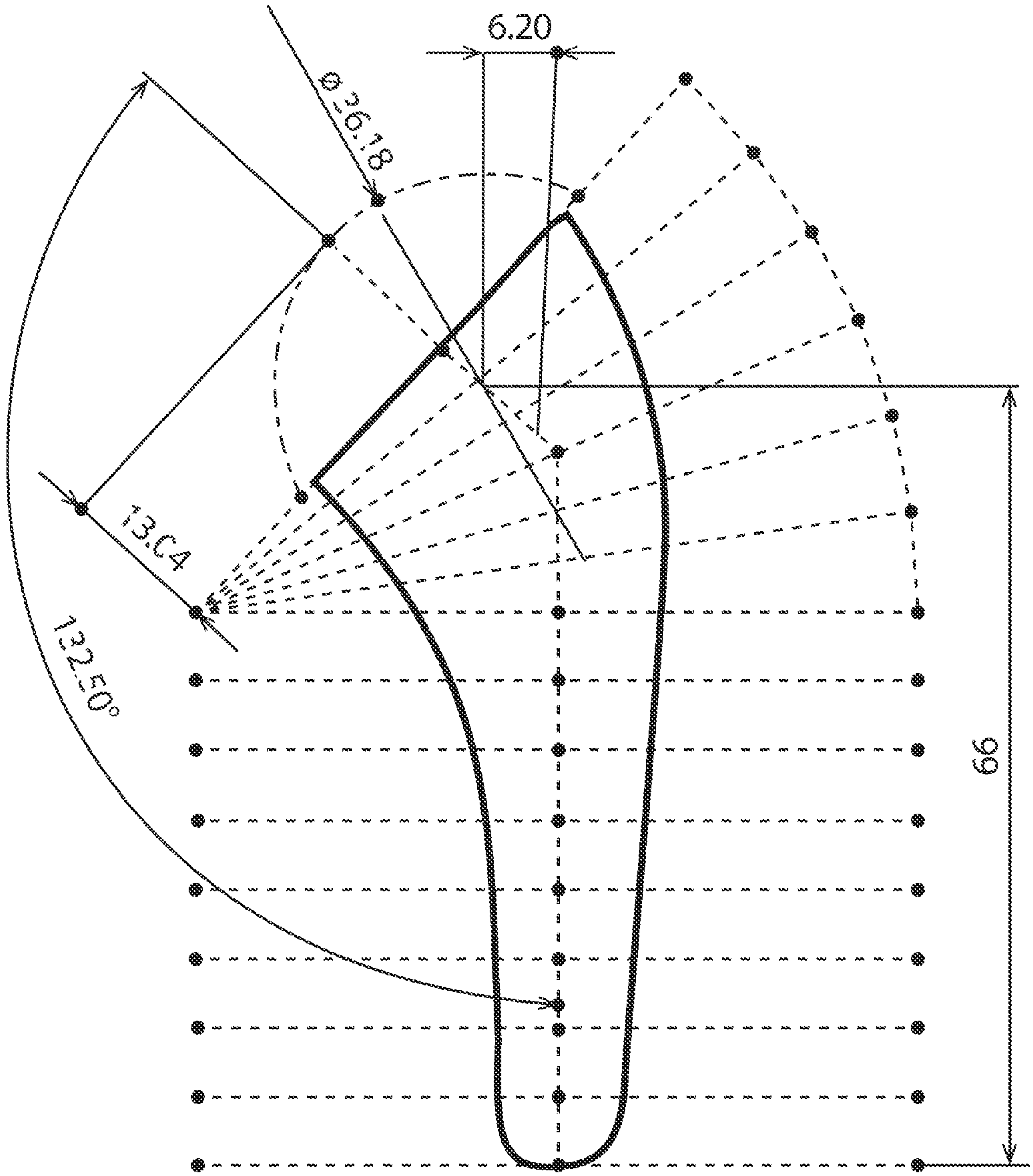


Fig. 16a

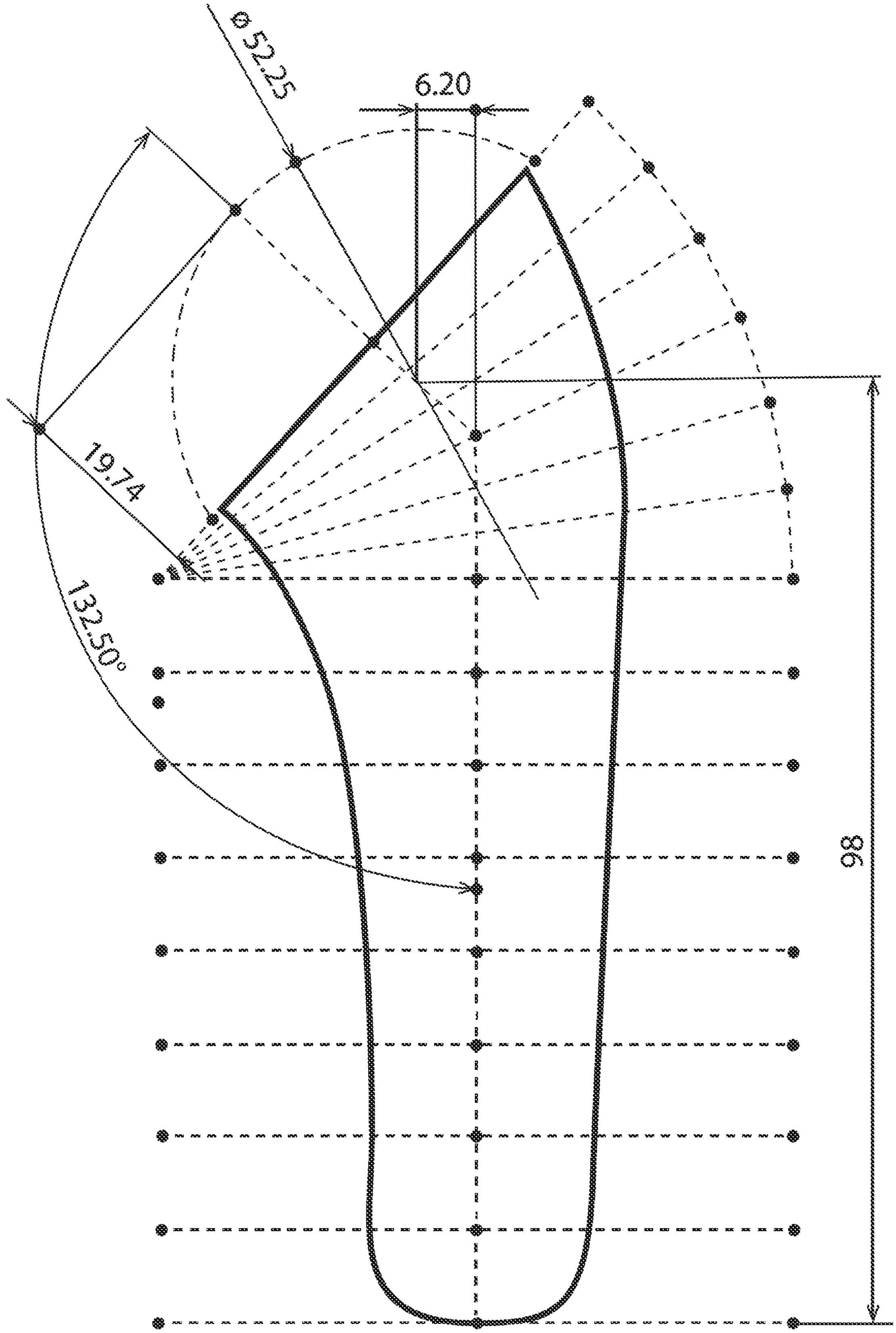


Fig. 16b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5725592 A
- US 20150250601 A